(19) Europäisches Patentamt
European Patent Office
Office européen des brevets

(11) Veröffentlichungsnummer: **0 014 469**
**A2**

# (12) EUROPÄISCHE PATENTANMELDUNG

(21) Anmeldenummer: 80100609.9

(22) Anmeldetag: 06.02.80

(51) Int. Cl.³: C 07 D 235/12
C 07 D 401/06, C 07 D 405/06
C 07 D 409/06, A 61 K 31/44
A 61 K 31/415, A 61 K 31/38
A 61 K 31/34

(30) Priorität: 09.02.79 CH 1291/79

(43) Veröffentlichungstag der Anmeldung: 20.08.80 Patentblatt 80/17

(84) Benannte Vertragsstaaten: BE CH DE FR GB IT LU NL SE

(71) Anmelder: CIBA-GEIGY AG
Patentabteilung Postfach
CH-4002 Basel(CH)

(72) Erfinder: Habicht, Ernst, Dr.
Bienenstrasse 13
CH-4104 Oberwil(CH)

(72) Erfinder: Ferrini, Pier Giorgio, Dr.
Im Rehwechsel 22
CH-4102 Binningen(CH)

(72) Erfinder: Sallmann, Alfred, Dr.
Joachimsackerstrasse 12
CH-4103 Bottmingen(CH)

(74) Vertreter: Zumstein sen., Fritz, Dr. et al,
Bräuhausstrasse 4
D-8000 München 2(DE)

## (54) Benzimidazol-2-derivate als Arzneimittel, ihre Verwendung und diese enthaltende pharmazeutische Präparate.

(57) Kernacylierte Benzimidazol-2-derivaten der Formel

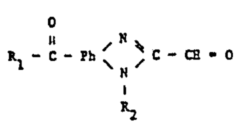

(I)

worin $R_1$ einen aliphatischen cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1$-C(=0)- enthaltende 1,2-Phenylengruppe darstellt, haben anti-allergische Eigenschaften, dienen als Arzneimittel und können als Arzneimittelwirkstoffe in pharmazeutischen Präparaten verwendet werden.

EP 0 014 469 A2

Croydon Printing Company Ltd.

CIBA-GEIGY AG
Basel (Schweiz)

4-12227/=

# Benzimidazol-2-derivate als Arzneimittel, ihre Verwendung und diese enthaltende pharmazeutischen Präparate

Die Erfindung betrifft kernacylierte Benzimidazol-2-derivaten der Formel

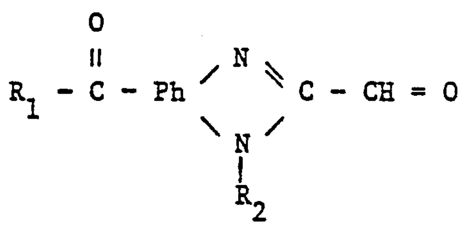

(I)

worin $R_1$ einen aliphatischen, cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1$-C(=O)- enthaltende 1,2-Phenylengruppe darstellt, als Arzneimittel, ihre Verwendung als Arzneimittelwirkstoff, sowie die Verbindungen der Formel I enthaltende pharmazeutische Präparate.

Im Zusammenhang mit der vorliegenden Beschreibung enthalten mit "nieder" bezeichnete, organische Reste und Verbindungen insbesondere bis und mit 7, vorzugsweise bis und mit 4 Kohlenstoffatome.

Aliphatische, cycloaliphatische, aromatische und araliphatische Reste $R_1$ bzw. $R_2$ sind in erster Linie gegebenenfalls substituierte aliphatische, cycloaliphatische, aromatische oder araliphatische Kohlenwasserstoffreste, wie entsprechendes Niederalkyl, Niederalkenyl, Cycloalkyl, Phenyl, Naphthyl oder Phenylniederalkyl. Substituenten sind z.B. Hydroxy, Niederalkoxy, Niederalkyl- oder Phenylthio, Niederalkyl- oder Phenylsulfinyl, oder Niederalkyl- oder Phenylsulfonyl, insbesondere von Niederalkyl $R_1$ sowie Niederalkyl $R_2$, ferner Niederalkyl, Niederalkoxy und/oder Halogen, insbesondere von Phenyl oder Phenylniederalkyl $R_1$. Heterocyclyl in einem heterocyclischen oder heterocyclisch-aliphatischen Rest $R_1$ ist in erster Linie monocyclisches Heterocyclyl aromatischen Charakters mit einem Heteroatom, wie Sauerstoff, Schwefel oder Stickstoff, als Ringglied, wie Furyl, Thienyl oder Pyridyl. In einem heterocyclisch-aliphatischen Rest $R_1$ ist der aliphatische Teil z.B. ein entsprechender aliphatischer Kohlenwasserstoffrest, insbesondere Niederalkyl.

Ausser durch den Rest der Formel $R_1$-C(=O) kann 1,2 Phenylen zusätzlich, u.a. durch Niederalkyl, Niederalkoxy, Hydroxy und/oder Halogen, einfach oder mehrfach substituiert sein.

Niederalkoxy bedeutet z.B. Methoxy, Aethoxy, n-Propyloxy, Isopropyloxy, Butyloxy, Isobutyloxy, tert.-Butyloxy, n-Pentyloxy oder n-Hexyloxy.

Phenylniederalkoxy ist z.B. Benzyloxy oder 1- oder 2-Phenyläthoxy.

Hydroxy-, Niederalkoxy- bzw. Diniederalkylaminoniederalkoxy ist insbesondere 2- und/oder 3-Hydroxy-niederalkoxy, z.B. 2-Hydroxyäthoxy, 3-Hydroxypropyloxy oder 2,3-Dihydroxy-propyloxy, sowie 2- oder 3-Niederalkoxy-niederalkoxy, z.B. 2-Methoxyäthoy, 2-Aethoxyäthoxy oder 3-Methoxypropyloxy, bzw. Diniederalkylaminoniederalkoxy, z.B. Dimethylamino- oder Diäthylaminoäthoxy.

Niederalkyl ist z.B. Methyl, Aethyl, n-Propyl, Isopropyl, N-Butyl, Isobutyl, tetr.-Butyl, n-Pentyl, n-Hexyl oder n-Heptyl.

Halogen ist insbesondere Halogen mit Atomnummer bis und mit 35, d.h. Fluor, Chlor oder Brom.

Niederalkylen ist z.B. 1,4-Butylen, 1,5-Pentylen oder 1,6-Hexylen.

Niederalkenyl ist z.B. Vinyl, 1-Methyl-vinyl, 1-Aethyl-vinyl, Allyl, 2- oder 3-Methyl-allyl oder 3,3-Dimethylallyl.

Cycloalkyl enthält vorzugsweise 3 bis 8 Ringatome und ist z.B. Cyclopropyl, Cyclopentyl, Cyclohexyl, Cycloheptyl oder Cyclooctyl.

Niederalkylthio ist z.B. Methylthio oder Aethylthio während Niederalkylsulfinyl und Niederalkylsulfonyl z.B. Methylsulfinyl, Aethylsulfinyl, Methylsulfonyl oder Aethylsulfonyl bedeuten.

Durch Niederalkylthio, Niederalkylsulfinyl oder Niederalkylsulfonyl substituiertes Niederalkyl ist z.B. Methylthio- oder Aethylthiomethyl, 1- oder 2-Methylthio- oder 1- oder 2-Aethylthioäthyl, oder 2- oder 3-Methylthio- oder 2- oder 3-Aethylthiopropyl, Methylsulfinyl- oder Aethylsulfinylmethyl, 1- oder 2-Methylsulfinyl- oder 1- oder 2-Aethylsulfinyl-äthyl, oder 2- oder 3-Methylsulfinyl- oder 2- oder 3-Aethylsulfinylpropyl, oder Methylsulfonyl- oder Aethylsulfonylmethyl, 1- oder 2-Methylsulfonyl- oder 1- oder 2-Aethylsulfonyläthyl, oder 2- oder 3-Methylsulfonyl- oder 2- oder 3-Aethylsulfonylpropyl. Durch Phenylthio, Phenylsulfinyl oder Phenylsulfonyl substituiertes Niederalkyl ist z.B. Phenylthio-, Phenylsulfinyl- oder Phenylsulfonylmethyl, oder 1- oder 2-Phenylthio-, 1- oder 2-Phenylsulfinyl-, oder 1- oder 2-Phenylsulfonyläthyl.

Phenylniederalkyl ist z.B. Benzyl, 1- oder 2-Phenyläthyl oder 1-, 2- oder 3-Phenylpropyl.

Furyl ist z.B. 2-Furyl, und Thienyl z.B. 2-Thienyl, während Pyridyl 2-, 3- oder 4-Pyridyl sein kann.

Furylniederalkyl, Thienylniederalkyl und Pyridylniederalkyl sind insbesondere entsprechend substituierte Methylreste, wie Furfuryl, 2-Thenyl oder Picolyl, z.B. 2- oder 4-Pyridylmethyl.

Die Verbindungen der Formel I zeigen wertvolle pharmakologische Eigenschaften. Insbesondere weisen sie antiallergische Wirkungen auf, die z.B. an der Ratte in Dosen von etwa 0,03 bis etwa 10 mg/kg bei intravenöser und in Dosen von etwa 1 bis etwa 100 mg/kg bei oraler Verabreichung im passiven kutanen Anaphylaxie-Test (PCA-Reaktion), der analog der von Goose und Blair, Immunology, Bd. 16, S. 794 (1969) beschriebenen Methode durchgeführt wird, nachgewiesen werden können, wobei die passive kutane Anaphylaxie nach dem von Ovary, Progr. Allergy, Bd. 5, S. 459 (1958), beschriebenen Verfahren erzeugt wird. Die antiallergische, insbesondere die degranulationshemmende Wirkung kann in einem in vitro-Versuch auch anhand der Histaminfreisetzung aus Peritonealzellen der Ratte im Dosisbereich von etwa 0,1 bis etwa 100 µg/ml bei immunologisch induzierter Freisetzung (wobei z.B. mit Nippostrongylus brasiliensis infestierte Ratten verwendet werden) und von etwa 1,0 bis etwa 100 µm/ml bei chemisch induzierter Freisetzung (wobei diese z.B. mit einem Polymeren von N-4-Methoxy-phenyläthyl-N-methyl-amin bewirkt wird) festgestellt werden. Die Verbindungen der vorliegenden Erfindung sind deshalb als Hemmer von allergischen Reaktionen, z.B. in der Behandlung und Prophylaxe von allergischen Erkrankungen, wie Asthma, sowohl extrinsic als auch intrinsic Asthma, oder anderen allergischen Erkrankungen, wie allergischer Rhinitis, z.B. Heufieber, Konjuktivitis, oder allergischer Dermatitis, z.B. Urticaria oder Ekzeme verwendbar.

Die Erfindung betrifft insbesondere Verbindungen der Formel I, worin $R_1$ gegebenenfalls durch Niederalkoxy, Niederalkylthio, Niederalkylsulfinyl, Niederalkylsulfonyl, Phenylthio, Phenylsulfinyl oder Phenylsulfonyl substituiertes Niederalkyl, Niederalkenyl, Cycloalkyl, gegebenenfalls im Phenylteil durch Niederalkyl, Niederalkoxy oder Halogen substituiertes Phenyl oder Phenylniederalkyl, Furyl, Thienyl oder Pyridyl, oder Furylniederalkyl, Thienylniederalkyl oder Pyridylniederalkyl bedeutet, $R_2$ Wasserstoff oder Niederalkyl darstellt, und Ph für den Rest der Formel $R_1$-C(=O)-enthaltendes und gegebenenfalls durch Niederalkyl, Niederalkoxy, Hydroxy und/oder Halogen substituiertes 1,2-Phenylen steht, als Arzneimittel, ihre Verwendung als Arzneimittelwirkstoff und diese enthaltende pharmazeutische Präparate.

Die Erfindung betrifft insbesondere Verbindungen der Formel I, worin $R_1$ Niederalkyl mit bis und mit 7 Kohlenstoffatomen, z.B. Methyl, Aethyl, n-Propyl, Isopropyl, n-Butyl, Isobutyl, tert.-Butyl, n-Pentyl, Neopentyl, n-Hexyl oder n-Heptyl, Niederalkoxy-, Niederalkylthio-, Niederalkylsulfinyl- oder Niederalkylsulfonyl-niederalkyl, worin die einzelnen Niederalkylreste bis und mit 4 Kohlenstoffatome enthalten, z.B. Methoxy-, Aethoxy-, Methylthio-, Aethylthio-, Methylsulfinyl-, Aethylsulfinyl-, Methylsulfonyl- oder Aethylsulfonylmethyl, 1- oder 2-Methoxy-, 1- oder 2-Aethoxy-, 1- oder 2-Methylthio-, 1- oder 2-Aethylthio-, 1- oder 2-Methylsulfinyl-, 1- oder 2-Aethylsulfinyl-, 1- oder 2-Methylsulfonyl- oder 1- oder 2-Aethylsulfonyläthyl, oder 1-, 2- oder 3-Methoxy-, 1-, 2- oder 3-Aethoxy-, 1-, 2- oder 3-Methylthio-, 1-, 2- oder 3-Aethylthio-,1-, 2- oder 3-Methylsulfinyl-, 1-, 2- oder 3-Aethylsulfinyl-, 1-, 2- oder 3-Methylsulfonyl- oder 1-, 2- oder 3-Aethylsulfonylpropyl, Phenylthio-, Phenylsulfinyl- oder Phenylsulfonyl-niederalkyl, worin der Niederalkylrest bis und mit 4 Kohlenstoffatome enthält, z.B. Phenylthio-, Phenylsulfinyl- oder Phenylsulfonylmethyl, 1- oder 2-Phenylthio-, 1- oder 2-Phenylsulfinyl- oder 1- oder 2-Phenylsulfonyläthyl, oder 1-, 2- oder 3-Phenylthio-, 1-, 2- oder 3-Phenylsulfinyl- oder 1-, 2- oder 3-Phenylsulfonylpropyl, Niederalkenyl mit bis und mit 5 Kohlenstoffatomen, z.B. 1-Methyl- oder 1-Aethyl-vinyl, oder Allyl, Cycloalkyl mit bis und mit 7 Kohlenstoffatomen, z.B. Cyclopropyl oder Cyclohexyl, gegebenenfalls durch Niederalkyl mit bis und mit 4 Kohlenstoffatomen, z.B. Methyl, Niederalkoxy mit bis und mit 4 Kohlenstoffatomen, z.B. Methoxy, und/oder Halogen mit Atomnummer bis und mit 35, z.B. Chlor oder Brom, substituiertes Phenyl oder Phenylniederalkyl mit bis und mit 4 Kohlenstoffatomen im Niederalkylrest, z.B. Benzyl oder 1- oder 2-Phenyläthyl, Furyl, Thienyl oder Pyridyl, z.B. 2-Furyl, 2-Thienyl oder 2-, 3- oder 4-Pyridyl, oder Furyl-, Thienyl- oder Pyridyl-niederalkyl mit bis und mit 4 Kohlenstoffatomen im Niederalkylrest, z.B. Furfuryl, 2-Thenyl oder 2- oder 4-Picolyl, bedeutet, $R_2$ Wasserstoff oder Niederalkyl mit bis und mit 4 Kohlenstoffatomen, z.B. Methyl, darstellt, und Ph das den Rest der Formel $R_1$-C(=O)- enthaltende, gegebenenfalls durch Niederalkyl mit bis und mit 4 Kohlenstoffatomen, z.B. Methyl, Niederalkoxy mit bis und mit 4 Kohlenstoffatomen, z.B. Methoxy, Hydroxy und/oder Halogen mit Atomnummer bis und mit 35, z.B. Chlor oder Brom, substituiertes 1,2-Phenylen darstellt, wobei der Rest der Formel $R_1$-C(=O)- irgendeine zur Substitution geeignete Stellung, vorzugsweise die 4- oder 5-Stellung des 1,2-Phenylenrestes einnimmt als Arzneimittel, ihre Verwendung als Arzneimittelwirkstoff und diese enthaltende pharmazeutische Präparate.

Die Erfindung betrifft in erster Linie Verbindungen der Formel

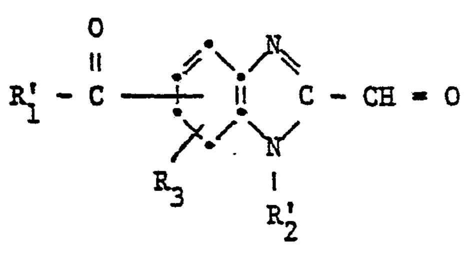

(Ia),

worin $R_1'$ insbesondere Niederalkyl mit bis und mit 7 Kohlenstoffatomen, z.B. Methyl, Aethyl, n-Propyl, n-Butyl, tert.-Butyl, n-Pentyl, n-Hexyl oder n-Heptyl, ferner Niederalkoxyniederalkyl, Niederalkylthioniederalkyl, Niederalkylsulfinylniederalkyl, Phenylthioniederalkyl oder Phenylsulfinylniederalkyl, worin die Niederalkylreste bis und mit 4 Kohlenstoffatome enthalten, z.B. Methoxy-, Methylthio-, Methylsulfinyl-, Phenylthio- oder Phenylsulfinylmethyl, 2-Methoxy-, 2-Methylthio-, 2-Methylsulfinyl-, 2-Phenylthio- oder 2-Phenylsulfinyläthyl, oder 3-Methoxy-, 3-Methylthio-, 3-Methylsulfinyl-, 3-Phenylthio- oder 3-Phenylsulfinyl-propyl, Cycloalkyl mit bis und mit 6 Ringkohlenstoffatomen, z.B. Cyclopropyl oder Cyclohexyl, Phenyl, Furyl oder Pyridyl, z.B. 3- oder 4-Pyridyl, darstellt, $R_2'$ für Wasserstoff oder Niederalkyl mit bis zu 4 Kohlenstoffatomen, z.B. Methyl, steht, und $R_3$ Wasserstoff, Niederalkyl mit bis und mit 4 Kohlenstoffatomen, z.B. Methyl, Niederalkoxy mit bis und mit 4 Kohlenstoffatomen, z.B. Methoxy, Hydroxy oder Halogen mit Atomnummer bis und mit 35, z.B. Chlor, bedeutet, wobei der Rest der Formel $R_1'-C(=O)-$ und die Gruppe $R_3$, falls diese von Wasserstoff verschieden ist, irgendeine der zur Substitution geeigneten, vorzugsweise die 5- und die 6-Stellung des Benzimidazolringes, einnehmen können, als Arzneimittel, ihre Verwendung als Arzneimittelwirkstoff und diese enthaltende pharmazeutische Präparate.

Die Erfindung betrifft in erster Linie Verbindungen der Formel Ia, worin $R_1'$ Niederalkyl mit bis und mit 7, z.B. mit bis und mit 4 Kohlenstoffatomen, z.B. Methyl, Aethyl, n-Propyl, Isopropyl oder n-Butyl, darstellt und $R_2'$, $R_3$ Niederalkyl mit bis und mit 4 Kohlenstoffatomen, z.B. Methyl, darstellen, wobei der Rest $R_1'-C(=O)-$ die 5- und der Niederalkylrest $R_3$ die 6-Stellung des Benzimidazolrings einnimmt, als Arzneimittel, ihre Verwendung als Arzneimittelwirkstoff und diese enthaltende pharmazeutische Präparate.

Die Erfindung betrifft namentlich die Verwendung der in den Beispielen genannten Verbindungen der Formel I und diese enthaltende pharmateutische Präparate.

Die Verbindungen der Formel I können in an sich bekannter Weise hergestellt werden. So kann man sie z.B. erhalten, indem man in einer Verbindung der Formel II

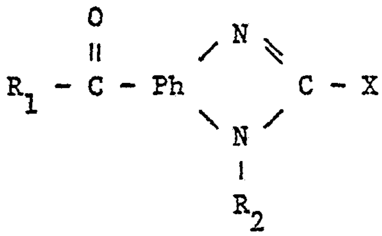

(II)

worin X einen oxidativ oder hydrolytisch in die Formylgruppe überführbaren Rest bedeutet, X oxidativ oder hydrolytisch in Formyl überführt.

Ein oxidativ in Formyl überführbarer Rest ist insbesondere die Hydroxymethylgruppe und eine hydrolytisch in Formyl überführbarer Rest vor allem eine acylalisierte oder acetalisierte Formylgruppe, wie verestertes oder veräthertes Dihydroxymethyl, z.B. Dihalogen-, wie Dichlormethyl, oder Niederalkylendioxy- oder Diniederalkoxymethyl, wie Propylendioxy- oder Diäthoxy- bzw. Dimethoxymethyl.

Die Oxidation erfolgt vorzugsweise durch Umsetzung mit Mangandioxid in Chloroform bei etwa 0 bis 50°C, vor allem bei etwa 10 bis 30°C. Die Hydrolyse einer acylalisierten Formylgruppe, beispielsweise von Dichlormethyl wird vorzugsweise in wässrigem Aethanol in Gegenwart von Natriumacetat bei etwa 40 bis 120°, vor allem 60-100°C, vorgenommen, während eine acetalisierte Formylgruppe vorzugsweise in wässrigem Dioxan in Gegenwart verdünnter Salzsäure hydrolysiert wird.

Die als Ausgangsstoffe zu verwendenden Verbindungen der Formel II können beispielsweise erhalten werden, indem man eine Verbindung der Formel

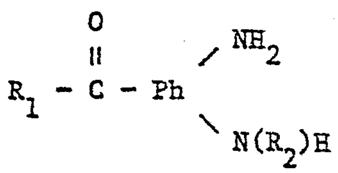

(III)

oder ein Salz, wie das Hydrochlorid davon mit einer Verbindung der Formel

$X - COOH$ (IV),

worin X Hydroxymethyl oder eine acylalisierte Formylgruppe, z.B. eine

Dihalogenmethylgruppe, bedeutet, umsetzt und gewünschtenfalls in der erhaltenen Verbindung eine acylalisierte Formylgruppe X durch Umsetzung mit einem entsprechenden Alkalimetallalkoholat, z.B. mit Natriumäthanolat, in acetalisiertes Formyl überführt.

Die pharmazeutischen Präparate der vorliegenden Erfindung, welche Verbindungen der Formel I enthalten. Bei den erfindungsgemässen pharmazeutischen Präparaten handelt es sich um solche zur enteralen, wie oralen, nasalen oder rektalen, sowie parenteralen oder topischen Verabreichung an Warmblüter, welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Erfindungsgemässe pharmazeutische Präparate sind z.B. solche in Dosiseinheitsformen, wie Dragées, Tabletten, Kapseln oder Suppositorien, sowie Ampullen, ferner Inhalationspräparate, ferner topisch und lokal (z.B. zur Insufflation) verwendbare pharmazeutische Zubereitungen. Die neuen pharmazeutischen Präparate enthalten z.B. bis etwa 95%, feste Darreichungsformen wie Dragées, Tabletten, Kapseln, Suppositorien und Insufflationspräparate beispielsweise von etwa 5 % bis etwa 90 %, insbesondere von etwa 10 bis 60 %, und Injektionsampullen sowie Inhalationspräparate beispielsweise von etwa 0,5 % bis etwa 20 %, insbesondere von etwa 1 % bis 10 %, des Wirkstoffes

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet. Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen- Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée -Ueberzügen können Farbstoffe oder Pigmente, z,B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten.

In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride Paraffinköhlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Inhalationspräparate für die Behandlung der Atemwege durch nasale oder buccale Verabreichung sind z.B. Aerosole Sprays oder Insufflationskapseln, welche den pharmakologischen Wirkstoff in Form eines Puders oder in Form von Tropfen einer Lösung oder Suspension verteilen können. Präparate mit Puder-verteilenden Eigenschaften enthalten ausser dem Wirkstoff üblicherweise ein flüssiges Treibgas mit einem Siedepunkt unter der Raumtemperatur, sowie, wenn erwünscht, Trägerstoffe, wie flüssige oder feste nicht-ionische oder anionische oberflächenaktive Mittel und/oder feste Verdünnungsmittel. Präparate, in welchen der pharmakologische Wirkstoff in Lösung vorliegt, enthalten ausser diesem ein geeignetes Treibmittel, ferner, falls notwendig, ein zusätzliches Lösungsmittel und/oder einen Stabilisator. Anstelle des Treibgases kann auch Druckluft verwendet werden, wobei diese mittels einer geeigneten Verdichtungs- und Entspannungsvorrichtung

nach Bedarf erzeugt werden kann.

Pharmazeutische Präparate für topische und lokale Verwendung sind z.B. für die Behandlung der Haut Lotionen und Crèmen, die eine flüssige oder semifeste Oel-in-Wasser- oder Wasser-in-Oel-Emulsion enthalten, und Salben (wobei solche vorzugsweise ein Konservierungsmittel enthalten), für die Behandlung der Augen Augentropfen, welche die aktive Verbindung in wässriger oder oliger Lösung enthalten und Augensalben, die vorzugsweise in steriler Form hergestellt werden, für die Behandlung der Nase Puder, Aerosole und Sprays (ähnlich den oben beschriebenen für die Behandlung der Atemwege), sowie grobe Puder, die durch schnelles Inhalieren durch die Nasenlöcher verabreicht werden, und Nasentropfen, welche die aktive Verbindung in wässriger oder öliger Lösung enthalten, oder für die lokale Behandlung des Mundes Lutschbonbons, welche die aktive Verbindung in einer im allgemeinen aus Zucker und Gummiarabikum oder Tragankanth gebildeten Masse enthalten, welcher Geschmacksstoffe beigegeben sein können, sowie Pastillen, die den Aktivstoff in einer inerten Masse, z.B. aus Gelatine und Glycerin oder Zucker und Gummiarabikum, enthalten.

Die Erfindung betrifft ebenfalls die Verwendung der neuen Verbindungen der Formel I oder Salzen davon als pharmakologisch aktive Verbindungen, insbesondere als Antiallergika, vorzugsweise in der Form von pharmazeutischen Präparaten. Die tägliche Dosis, die einem Warmblüter von etwa 70 kg verabreicht wird, beträgt, ist abhängig von der Applikationsform. Bei oraler Application werden Tagesdosen von etwa 50 bis etwa 500 mg, vorzugsweise von etwa 75 bis 250 mg, gewünschtenfalls in mehreren Einzeldosen über den Tag verteilt, z.B. in 1 bis 4 Einzeldosen, empfohlen.

Die nachfolgenden Beispiele illustrieren die oben beschriebene Erfindung; sie sollen jedoch den Erfindungsumfang in keiner Weise einschränken. Terperaturen werden in Celsiusgraden angegeben.

Beispiel 1: 25 g Mangandioxid werden in 200 ml alkoholfreiem Chloroform 2 Stunden durch azeotropes Entwässern aktiviert. Man kühlt unter Rühren auf 10°C ab, gibt 4,92 g 5-Butyryl-1,6-dimethyl-benzimidazol-2-methanol hinzu, lässt langsam auf Raumtemperatur erwärmen und rührt 18 Stunden bei Raumtemperatur. Man filtriert ab, dampft das Filtrat zur Trockne ein und kristallisiert den Eindampfrückstand aus Chloroform/Petroläther um. Man erhält den 5-Butyryl-1,6-dimethyl-benzimidazol-2-carboxaldehyd vom Smp. 116,5-118°,

Das Ausgangsmaterial kann z.B. folgendermasser hergestellt werden:

Ein Gemisch von 24,1 g 4-Chlor-2-methyl-5-nitro-butyrophenon und 250 ml einer 33%-igen Lösung von Methylamin in Aethanol wird bei Raumtemperatur stehengelassen; das kristalline Ausgangsmaterial löst sich langsam auf, wobei Gelbfärbung eintritt. Die Reaktion ist schwach-exotherm; man kühlt deshalb mit einem Wasserbad, un ein zu starkes Entweichen von Methylamin zu verhindern. Nach 20 Minuten tritt vollständige Lösung ein,dann beginnt ein Niederschlag auszufallen. Man lässt während 16 Stunden bei Raumtemperatur stehen und dampft dann unter vermindertem Druck zur Trockne ein. Der Rückstand wird mit Diäthyläther (etwa 1000 ml), Eis und Natriumcarbonat versetzt, durchgeschüttelt und die organische Schicht abgetrennt. Diese wird zweimal mit Wasser gewaschen und die wässrige Lösung mit Diäthyläther zurückgewaschen. Die vereinigten organischen Lösungen werden über Natriumsulfat getrocknet, filtriert und auf ein Volumen von etwa 300 ml eingedampft, dann mit 100 ml Petroläther verdünnt und gekühlt. Das gelbe, kristalline 2-Methyl-4-methylamino-5-nitro-butyrophenon fällt aus, wird abfiltriert, mit Petroläther gewaschen und an der Luft getrocknet, F. 107-108°.

Eine Lösung von 4,7 g 2-Methyl-4-methylamino-4-nitro-butyrophenon in 40 ml Dioxan wird mit Wasser verdünnt und zum Rückfluss erhitzt, dann innerhalb von 10 Minuten mit einer Lösung von 16 g

Natriumdithionit in 70 ml Wasser behandelt, wobei die gelbe Farbe des Reaktionsgemisches verblasst. Man kocht während 15 Minuten am Rückfluss weiter, stellt den pH-Wert durch Zugabe von etwa 30 ml 6-n. Salzsäure auf 3 ein und kocht nochmals 15 Minuten unter Rückfluss, während welcher Zeit das Schwefeldioxid entweicht. Der pH-Wert des Reaktionsgemisches wird auf 2 gestellt, nochmals während etwa 5 Minuten unter Rückfluss gekocht und das Dioxan dann unter vermindertem Druck verdampft. Aus der zurückbleibenden Lösung fällt das Hydrochlorid des 5-Amino-2-methyl-4-methylamino-butyrophenons aus; man kühlt die Suspension, stellt mit konzentrierter wässriger Natriumhydroxidlösung alkalisch und extrahiert mit Chloroform. Der organische Extrakt wird zweimal mit Wasser gewaschen, getrocknet, filtriert und eingedampft. Das 5-Amino-2-methyl-4-methylamino-butyrophenon schmilzt bei 126-128°.

Die Ueberführung von 4-Chlor-2-methyl-5-nitro-butyrophenon in 4-Amino-2-methyl-5-methylamino-butyrophenon kann auch folgendermassen durchgeführt werden, wobei man auch von einem rohen Isomerengemisch ausgehen kann.

241 g rohes Chlor-methyl-nitrobutyrophenon (etwa 70%-ig an 4-Chlor-2-methyl-5-nitro-butyrophenon) werden in 1200 ml Aethanol suspendiert und mit 1200 ml 33%-iger Methylaminlösung versetzt, worauf unter exothermer Reaktion Auflösung erfolgt. Man lässt 2 Tage stehen, dampft unter vermindertem Druck zur Trockne ein, versetzt mit 100 ml 2n-Salzsäure und erwärmt 1 Stunde auf 80-90°. Man kühlt durch Zugabe von Eis auf etwa 15° ab, saugt den kristallinen Niederschlag ab, wäscht mit Wasser nach, nimmt in Methylenchlorid auf, trocknet über Natriumsulfat, dampft unter vermindertem Druck, zuletzt unter Zugabe von Cyclohexan und Petroläther (Siedebereich 60-80°), das Methylenchlorid ab, kühlt und saugt das 2-Methyl-4-methylamino-5-nitrobutyrophenon vom Smp. 105-107° ab.

59,1 g 2-Methyl-4-methylamino-5-nitro-butyrophenon werden in 1000 ml Methanol gelöst, mit 6 g Raney-Nickel versetzt und bei 20-25° unter Normaldruck hydriert. Nach Aufnahme von 16,8 Litern Wasserstoff wird die Hydrierung abgebrochen, zur Auflösung des Ausgefallenen gelinde erwärmt, vom Katalysator abfiltriert, mit 50 ml konzentrierter Salzsäure versetzt, auf 3° abgekühlt und abgesaugt, Man erhält das 4-Amino-2-methyl-5-methylamino-butyrophenon-hydrochlorid vom Smp. über 180° (Zers.).

Ein Gemisch von 9,8 g 5-Amino-2-methyl-4-methylamino-butyrophenon und 4,15 g Glycolsäure wird in einem Oelbad von 130°C erhitzt. Nach 150 Minuten wird das Reaktionsprodukt, zusammen mit dem Produkt eines zweiten Ansatzes von 3 g 5-Amino-2-methyl-4-methylamino-butyrophenon und 1,27 g Glycolsäure, in 300 ml 2-n. Salzsäure aufgenommen und filtriert. Das Filtrat wird alkalisch gestellt; man extrahiert das ausgefallene Oel mit drei Portionen Essigsäureäthylester, wäscht den organischen Extrakt zweimal mit Wasser, trocknet, filtriert und dampft ein. Der braune, ölige Rückstand kristallisiert spontan und wird aus Essigsäureäthylester umkristallisiert. Das so erhältliche 5-Butyryl-1,6-dimethyl-benzimidazol-2-methanol schmilzt bei 141,5-142,5°.

<u>Beispiel 2:</u> 2,0 g 5-Butyryl-2-dichlormethyl-1,6-dimethylbenzimidazol werden in 100 ml Aethanol gelöst. Die Lösung wird mit 50 ml einer 4n-Natriumacetatlösung versetzt und 5 Stunden zum Rückfluss erhitzt. Man dampft unter vermindertem Druck zur Trockne ein und kristallisiert aus Essigsäureäthylester/Cyclohexan um. Man erhält den 5-Butyryl-1,6-dimethylbenzimidazol-2-carboxaldehyd vom Smp. 116-118°.

Das Ausgangsmaterial kann z.B. folgendermassen erhalten werden:

2,06 g 3-Amino-2-methyl-4-methylamino-butyrophenon werden in 40 ml 6n-Salzsäure gelöst, mit 2,6 g Dichloressigsäure versetzt und 5 Stunden zum Rückfluss erhitzt. Man lässt abkühlen, verdünnt mit

Wasser und extrahiert mit Methylenchlorid. Beim Eindampfen des Extraktes hinterbleibt das 5-Butyryl-2-dichlormethyl-1,6-dimethyl-benzimidazol in Form einer honigartigen Masse.

Beispiel 3: In analoger Weise wie in Beispiel 1 und 2 beschrieben kann man ferner herstellen:

5-Acetyl-1-methyl-benzimidazol-2-carboxaldehyd,
5-Butyryl-1-methyl-benzimidazol-2-carboxaldehyd,
1,6-Dimethyl-5-valeryl-benzimidazol-2-carboxaldehyd,
1-Aethyl-5-butyryl-6-methyl-benzimidazol-2-carboxaldehyd,
5-Acetyl-1-butyl-benzimidazol-2-carboxaldehyd,
1-Butyryl-5-butyryl-6-methyl-benzimidazol-carboxaldehyd,
5-Cyclopropylcarbonyl-1,6-dimethyl-benzimidazol-2-carboxaldehyd,
5-Butyryl-6-methyl-benzimidazol-2-carboxaldehyd,
5(6)-Valeroyl-benzimidazol-2-carboxaldehyd,
5-Valeroyl-6-methyl-benzimidazol-2-carboxaldehyd,
5(6)-Butyryl-benzimidazol-2-carboxaldehyd,
5-Butyryl-6-methoxy-benzimidazol-2-carboxaldehyd,
5-Butyryl-6-chlor-benzimidazol-2-carboxaldehyd,
5-Acetyl-6-methyl-benzimidazol-2-carboxaldehyd,
5-Propionyl-6-methyl-benzimidazol-2-carboxaldehyd,
5-Cyclohexylcarbonyl-6-methyl-benzimidazol-2-carboxaldehyd,
5-(4-Methoxybutyryl)-6-methyl-benzimidazol-2-carboxaldehyd,
5-(4-Methylthiobutyryl)-6-methyl-benzimidazol-2-carboxaldehyd,
5-(4-Methylsulfinylthiobutyryl)-6-methyl-benzimidazol-2-carboxaldehyd,
5-(4-Phenylthiobutyryl)-6-methyl-benzimidazol-2-carboxaldehyd,
5-(4-Phenylsulfinylbutyryl)-6-methyl-benzimidazol-2-carboxaldehyd,
5-Propionyl-1,6-dimethyl-benzimidazol-2-carboxaldehyd und
5-Isobutyryl-1,6-dimethyl-benzimidazol-2-carboxaldehyd.

Beispiel 4: Eine zur Inhalation geeignete, 2%-ige wässrige Lösung von 5-Butyryl-1,6-dimethyl-benzimidazol-2-carboxaldehyd kann wie folgt hergestellt werden:

Zusammensetzung (für 100 ml)

| | |
|---|---|
| 5-Butyryl-1,6-dimethyl-benzimidazol-2-carboxaldehyd | 2.000 g |
| Dinatriumsalz der Aethylendiamin-tetraessigsäure (Stabilisator) | 0.010 g |
| Benzalkoniumchlorid (Konservierungsmittel) | 0.010 g |
| Wasser, destilliert | ad 100 ml |

Der 5-Butyryl-1,6-dimethyl-benzimidazol-2-carboxaldehyd wird unter Zugabe eines Lösungsvermittlers, z.B. Polyäthylenglykol in frisch destilliertem Wasser gelöst, und die Lösung mit dem Dinatriumsalz der Aethendiamin-tetraessigsäure und dem Benzalkoniumchlorid (Gemisch von Alkyl-dimethyl-benzyl-ammoniumchloride, worin Alkyl von 8 bis 18 Kohlenstoffatome enthält) versetzt. Nach völlständiger Auflösung der Komponenten wird die erhaltene Lösung mit Wasser auf ein Volumen von 100 ml gebracht, abgefüllt und gasdicht verschlossen.

Beispiel 5: Zur Insufflation geeignete, 0,025 g der 5-Butyryl-1,6-dimethyl-benzimidazol-2-carboxaldehyd enthaltende Kapseln können wie folgt hergestellt werden:

Zusammensetzung (für 1000 Kapseln)

| | |
|---|---|
| 5-Butyryl-1,6-dimethyl-benzimidazol-2-carboxaldehyd | 25.00 g |
| Lactose, gemahlen | 25.00 g |

Die 5-Butyryl-1,6-dimethyl-benzimidazol-2-carboxaldehyd und die Lactose (feinst gemahlen) werden gut miteinander vermischt. Das erhaltene Pulver wird dann gesiebt und die Portionen zu je 0,05 g in Gelatinekapseln abgefüllt.

Beispiel 6: Tabletten enthaltend 100 mg 5-Butyryl-1,6-dimethyl-benzimidazol-2-carboxaldehyd (Wirkstoff) können beispielsweise in folgender Zusammensetzung hergestellt werden:

| Zusammensetzung | Pro Tablette |
| --- | --- |
| Wirkstoff, z.B. 5-Butyryl-1,6-dimethyl-benzimidazol-2-carboxaldehyd | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

Herstellung

Der Wirkstoff wird mit dem Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister abgeknetet, bis eine schwach plastische Masse entstanden ist. Die Masse wird durch ein Sieb von ca. 3 mm Maschenweite getrieben, getrocknet und das trockene Granulat nochmals durch ein Sieb getrieben. Darauf werden die restliche Weizenstärke, Talk und Magnesiumstearat zugemischt. Die erhaltene Mischung wird zu Tabletten von 250 mg mit Bruchkerbe(n) verpresst.

Beispiel 7: In analoger Weise wie in den Beispielen 4 bis 6 beschrieben kann man pharmazeutische Präparate enthaltend eine Verbindung gemäss einen der Ansprüche 2 und 3 herstellen.

Patentansprüche

1. Kernacylierte Benzimidazol-2-derivate der Formel

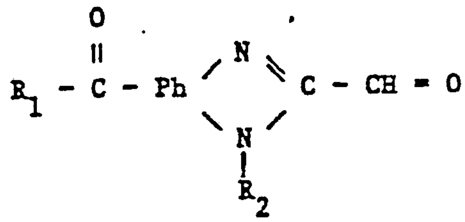

(I)

worin R eine gegebenenfalls veresterte oder amidierte Carboxygruppe oder eine gegebenenfalls verätherte oder veresterte Hydroxymethylgruppe bedeutet, $R_1$ einen aliphatischen, cycloaliphatischen, aromatischen, araliphatischen, heterocyclischen oder heterocyclisch-aliphatischen Rest darstellt, $R_2$ für Wasserstoff oder einen aliphatischen Rest steht, und Ph eine den Rest $R_1$-C(=O)- enthaltende 1,2-Phenylengruppe darstellt, zur prophylaktischen oder therapeutischen Behandlung des menschlichen oder tierischen Körpers.

2. Verbindungen gemäss Anspruch 1 der Formel I, worin $R_1$ Niederalkyl mit bis und mit 7 Kohlenstoffatomen, Niederalkoxy-, Niederaklylthio-, Niederalkylsulfinyl- oder Niederalkylsulfonyl-niederalkyl, worin die einzelnen Niederalkylreste bis und mit 4 Kohlenstoffatome enthalten, Phenylthio-, Phenylsulfinyl- oder Phenylsulfonyl-niederalkyl, worin der Niederalkylrest bis und mit 4 Kohlenstoffatome enthält, Niederalkenyl mit bis und mit 5 Kohlenstoffatomen, Cycloalkyl mit bis und mit 7 Kohlenstoffatomen, gegebenenfalls durch Niederalkyl mit bis und mit 4 Kohlenstoffatomen, Niederalkoxy mit bis und mit 4 Kohlenstoffatomen, und/oder Halogen mit Atomnummer bis und mit 35 substituiertes Phenyl oder Phenylniederalkyl mit bis und mit 4 Kohlenstoffatomen im Niederalkylrest, Furyl, Thienyl oder Pyridyl, oder Furyl-, Thienyl- oder Pyridylniederalkyl mit bis und mit 4 Kohlenstoffatomen im Niederalkylrest bedeutet, $R_2$ Wasserstoff oder Niederalkyl mit bis und mit 4 Kohlenstoffatomen darstellt, und Ph das den Rest der Formel $R_1$-C(=O)- enthaltende,

gegebenenfalls durch Niederalkyl mit bis zu und mit 4 Kohlenstoffatomen, Niederalkoxy mit bis und mit 4 Kohlenstoffatomen, Hydroxy und/oder Halogen mit Atomnummer bis und mit 35 substituiertes 1,2-Phenylen darstellt, zur porphylaktischen oder therapeutischen Behandlung des menschlichen oder tierischen Körpers.

3. Verbindungen gemäss Anspruch 1 der Formel

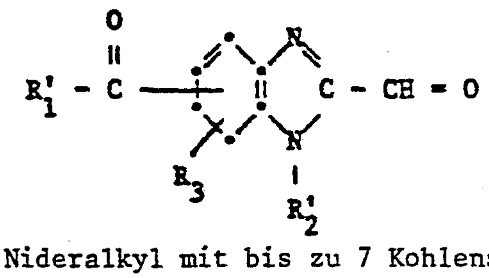

(Ia),

worin $R_1'$ Nideralkyl mit bis zu 7 Kohlenstoffatomen, Niederalkoxyniederalkyl, Niederalkylthioniederalkyl, Niederalkylsulfinylniederalkyl, Phenylthioniederalkyl oder Phenylsulfinylniederalkyl, worin die Niederalkylreste bis und mit 4 Kohlenstoffatome enthalten, Cycloalkyl mit bis und mit 6 Ringkohlenstoffatomen, Phenyl, Furyl oder Pyridyl darstellt, $R_2'$ für Wasserstoff oder Niederalkyl mit bis und mit 4 Kohlenstoffatomen steht, und $R_3$ Wasserstoff, Niederalkyl mit bis und mit 4 Kohlenstoffatomen, Niederalkoxy mit bis und mit 4 Kohlenstoffatomen, Hydroxy oder Halogen mit Atomnummer bis und mit 35 bedeutet, zur prophylaktischen oder therapeutischen Behandlung des menschlichen oder tierischen Körpers.

4. Verbindungen gemäss Anspruch 3 der Formel Ia, worin $R_1'$ Niederalkyl mit bis und mit 7 Kohlenstoffatomen, Cycloalkyl mit bis und mit 6 Ringkohlenstoffatomen oder Phenyl bedeutet, $R_2'$ für Wasserstoff oder Niederalkyl mit bis und mit 4 Kohlenstoffatomen steht, und $R_3$ Wasserstoff, Niederalkyl mit bis und mit 4 Kohlenstoffatomen, Niederalkoxy mit bis und mit 4 Kohlenstoffatomen oder Halogen mit Atomnummer bis und mit 35 bedeutet, als Arzneimittel, zur prophylaktischen oder therapeutischen Behandlung des menschlichen oder tierischen Körpers.

5. Verbindungen gemäss Anspruch 3 der Formel Ia, worin $R_1'$ Niederalkyl mit bis und mit 7 Kohlenstoffatomen darstellt, und $R_2'$ und $R_3$ Niederalkyl mit bis und mit 4 Kohlenstoffatomen darstellt, wobei

der Rest $R_1'$-C(=O)- die 5- und der Niederalkylrest $R_3$ die 6-Stellung des Benzimidazolrings einnimmt, zur prophylaktischen oder therapeutischen Behandlung des menschlichen oder tierischen Körpers.

6. 5-Butyryl-1,6-dimethyl-benzimidazol-2-carboxaldehyd zur prophylaktischen oder therapeutischen Behandlung des menschlichen oder tierischen Körpers.

7. 5(6)-Valeryl-benzimidazol-2-carboxaldehyd,
5-Butyryl-6-methyl-benzimidazol-2-carboxaldehyd,
5-Acetyl-6-methyl-benzimidazol-2-carboxaldehyd,
6-Methyl-5-propionyl-benzimidazol-2-carboxaldehyd,
6-Methyl-5-valeryl-benzimidazol-2-carboxaldehyd,
5(6)-Butyryl-benzimidazol-2-carboxaldehyd,
5-Butyryl-6-methoxy-benzimidazol-2-carboxaldehyd,
5-Butyryl-6-chlor-benzimidazol-2-carboxaldehyd,
5-Cyclopropylcarbonyl-1,6-methyl-benzimidazol-2-carboxaldehyd,
5-Cyclohexylcarbonyl-6-methyl-benzimidazol-2-carboxaldehyd,
5-(4-Methoxy-butyryl)-6-methyl-benzimidazol-2-carboxaldehyd,
6-Methyl-5-(4-methylthio-butyryl)-benzimidazol-2-carboxaldehyd,
6-Methyl-5-(4-methylsulfinyl-butyryl)-benzimidazol-2-carboxaldehyd,
6-Methyl-5-(4-phenylthio-butyryl)-benzimidazol-2-carboxaldehyd,
6-Methyl-5-(4-phenylsulfinyl-butyryl)-benzimidazol-2-carboxaldehyd,
5-Acetyl-1-methyl-benzimidazol-2-carboxaldehyd,
5-Butyryl-1-methyl-benzimidazol-2-carboxaldehyd,
1,6-Dimethyl-5-valeryl-benzimidazol-2-carboxaldehyd,
1-Aethyl-5-butyryl-6-methyl-benzimidazol-2-carboxaldehyd,
5-Acetyl-1-n-butyl-benzimidazol-2-carboxaldehyd,
1-n-Butyl-5-butyryl-6-methyl-benzimidazol-2-carboxaldehyd,
5-Propionyl-1,6-dimethyl-benzimidazol-2-carboxaldehyd oder
5-Isobutyryl-1,6-dimethyl-benzimidazol-2-carboxaldehyd zur prophylaktischen oder therapeutischen Behandlung des menschlichen oder tierischen Körpers.

8. Pharmazeutische Präparate enthaltend mindestens eine Verbindung gemäss einem der Ansprüche 1 - 7 neben üblichen pharmazeutischen Hilfs- und Trägerstoffen.

9. Oral applizierbare pharmazeutische Präparate gemäss Anspruch 8.

10. Verwendung einer Verbindung gemäss einem der Ansprüche 1 bis 7 als Arzneimittelwirkstoff in pharmazeutischen Präparaten.